# EUROPEAN PATENT APPLICATION

(11) **EP 4 667 002 A1**
(43) Date of publication of application: **24.12.2025**
(21) Application number: 24183758.2
(22) Date of filing: 21.06.2024
(51) Int. Cl.: A61K 9/20, B33Y 10/00, B33Y 30/00, B33Y 70/00, B33Y 80/00

(54) **3D PRINTING OF A TABLET COMPRISING AN ENTERIC COATING**

(71) Applicant: Nederlandse Organisatie voor toegepast-natuurwetenschappelijk Onderzoek TNO, 2595 DA 's-Gravenhage (NL)
(72) Inventor: VAN BOMMEL, Kjeld Jacobus Cornelis, 2595 DA `s-Gravenhage (NL); VAN EE, Renz Jeroen, 2595 DA `s-Gravenhage (NL)
(74) Representative: V.O.

(57) **Abstract**

A method for manufacturing a tablet, preferably a 3D printable pharmaceutical product (100). The method as described herein comprises repeated steps of: providing a layer of a homogeneous powder formulation (1), the formulation comprising a first excipient (2) and an active pharmaceutical ingredient (3). The tablet comprising a core and an enteric coating. Preferably the core comprises an active pharmaceutical ingredient (API).

## Description

### TECHNICAL FIELD OF THE INVENTION

The present invention relates to a method of manufacturing a tablet comprising an enteric coating and an active pharmaceutical ingredient. Preferably the method of manufacturing is 3D printing. The invention further discloses a pharmaceutical product.

### BACKGROUND OF THE INVENTION

At present, the healthcare industry is more and more considering to provide personalized healthcare. Personalized healthcare, often referred to as personalized medicine is defined by the European Union as "*A medical model using characterization of individuals' phenotypes and genotypes (e.g. molecular profiling, medical imaging, lifestyle data) for tailoring the right therapeutic strategy for the right person at the right time, and*/*or to determine the predisposition to disease and*/*or to deliver timely and targeted prevention*"*.* To transfer from the healthcare as we know today to personalized medicine, various research has been done on multiple topics. Particular attention has been given to the manufacturing of pharmaceutical products, which predominantly are produced en masse, with limited possibility to personalize. Therefore, multiple research projects have focused on providing new manufacturing technologies suitable for personalized medicine such as a personalized pharmaceutical product.

A promising technology to produce more personalized medicine may be 3D printing. 3D printing is known for being versatile and flexible such that it would be possible to easily manufacture a tablet according to the needs of a subject. Typical examples may be to change the shape of a tablet, the dosage of the active pharmaceutical ingredient in a tablet or the release rate of the active pharmaceutical ingredient. Typical examples of 3D printing technologies are fused deposition modelling, selective laser sintering and binder jet or powder bed printing.

Fused deposition modelling printers extrude a thermoplastic filament in a series of layers over a build plate to create a three-dimensional object.

Selective laser sintering is a 3D printing technology that uses a high-power laser to sinter small particles of polymer powder into a solid structure based on a 3D model.

3D printing technologies like Fused Deposition Modelling and Selective Lasering Sintering may have the disadvantage that high temperatures are needed to produce the pharmaceutical product in a manner that the API (and/or excipient) used in the pharmaceutical product may be at risk for degradation.

A possible alternative may be binder jetting or powder bed printing. In binder jetting or powder bed printing a powder comprising an API, and one or more excipients may be consolidated employing liquid droplets. The one or more excipients or API may be capable of at least partially dissolving into the liquid droplets such that the powder particles adhere to each other, forming a solidified layer.

Some APIs are sensitive to stomach conditions such that proper protection of the APIs is needed. Usually, APIs are most effective and efficient when they reach a specific location in the human body. As such, an API should be able to resist the severe conditions associated with the environment of the stomach such that the API is released after the stomach. Therefore, a tablet, that comprises an API sensitive to acidic conditions such as in a stomach, are often coated with an enteric coating such that the tablet is able to resist the conditions in the stomach. Typically, two different compositions, one for the coating and one for the tablet, are needed to produce an enteric coated tablet. Binder jetting or powder bed printing are traditionally not compatible with multiple powder compositions to produce a final enteric coated pharmaceutical product as achieving the required resolution for an enterically coated product is challenging. In order to manufacture a tablet such as a pharmaceutical product, in general two different compositions need to be designed to ensure that the API is released in the required area. The first composition may be related to a composition comprising the active pharmaceutical ingredient while the second composition may include the enteric compound.

A conventional process to produce an enteric coated tablet such as a pharmaceutical product, in general, comprises a two-step process, wherein a first process step involves compressing a pharmaceutical composition comprising the API to a tablet and a second processing step involves adding an enteric coating. Again, multiple different process steps are needed, possibly impacting to final cost of the pharmaceutical product and the investment cost, as at least 2 machines may be needed to produce a tablet.

A first and second example of a pharmaceutical product manufactured according to the aforementioned conventional method are described in the respective articles entitled: "Formulation of a 3D printed Biopharmaceutical: The Development of an Alkaline Phosphatase Containing Tablet with Ileo-Colonic Release Profile to Treat Ulcerative Colitis" and "Surface Engineering Methods for Powder Bed Printed Tablets to Optimize External Smoothness and Facilitate the Application of Different Coatings". The first article discloses a pharmaceutical product comprising ileo-colonic controlled release profile possibly suitable for clinical relevant application. The second article describes a pharmaceutical product comprising a relative smooth surface. The pharmaceutical product comprising a Powder Bed Printed tablet including the API ingredient and an enteric coating. Both articles describe a two-step process to manufacture a tablet such as a pharmaceutical product. Additionally, when employing a two-step process comprising powder bed printing as first step to manufacture a tablet, powder bed printing or 3D printing in general may negatively influence the smoothness of the tablet such that an enteric coating, applied during the second process step, may not be able to fully cover the tablet such that the API is not properly protected from the conditions present in the stomach.

The disclosed invention is inclined to solve at least one of the above-mentioned disadvantages like for example the exposure of materials to high temperatures and/or complex processing like for example the conventional two-step process to produce a coated tablet. Accordingly, the present invention is aimed at providing a method of manufacturing a tablet such as a pharmaceutical product comprising a single process or comprising a single powder formulation.

### SUMMARY

The present disclosure is related to a method for manufacturing a tablet, preferably a pharmaceutical product.

Accordingly, a method for manufacturing a 3D printable tablet, preferably a pharmaceutical product has been designed. The method comprises repeated steps of providing a layer of a homogeneous powder formulation, the formulation comprising a first excipient and an active pharmaceutical ingredient (API). The first excipient suitable for consolidation by a consolidation means of a first type and the active pharmaceutical ingredient preferably not suitable for consolidation by the consolidation means of the first type. After providing a layer of homogeneous powder, the method is continued by applying a consolidation means of a first type in a first pattern on the layer of homogeneous powder formulation. The 3D printable pharmaceutical product having an outside contour formed by the first pattern suitable for forming an enteric coating. The method may further comprise the repeated step of applying a consolidation means of a second type in a second pattern on the layer of homogeneous powder formulation.

In a preferred embodiment according to the invention, the method may further comprise providing a second excipient that may be suitable for consolidation means of the second type. In a further embodiment, the second excipient and/or the API may be suitable for consolidation by a consolidation means of a second type. Providing a second excipient may have the advantage that the consolidation of the consolidation means of the second type is independent from the API present in the homogeneous powder formulation.

In another embodiment the first and the second pattern may be complementary.

Having a consolidation means of the first type for the enteric coating and optionally a consolidation means of the second type for the API and/or second excipient, respectively, may have the advantage of utilizing one homogeneous powder formulation instead two or more different formulations as encountered in conventional two-step processes to manufacture a coated tablet. Another advantage may be that the tablet, preferably a pharmaceutical product, may be manufactured in a single process.

In a further embodiment according to the invention, the outside contour may act as an enteric coating. The outside contour as enteric coating may have a thickness of at least 0.05 mm, preferably at least 0.1 mm.

Ensuring an outside contour thickness of at least 0.05 mm, preferably at least 0.1 mm may have the advantage of easily producing an enteric coating that may fully encapsulate the core of a tablet such that the coated tablet would be able to resist acidic conditions.

In a further embodiment of the present invention, the 3D printable pharmaceutical product may be manufactured in a single process.

A single process may have the advantage of increasing the efficiency, reducing cost or potentially solve the issues encountered when separately coating a 3D printed tablet with an enteric coating such as in conventional two-step processes. Typical issues that may arise in a two-step process may be related to the limited coverage of the enteric coating to the tablet such that the API may prematurely dissolve such that the required area will not be reached.

In an even further embodiment according to the invention, the consolidation means of the first type may comprise a first printing liquid. The first printing liquid may be suitable for the consolidation of the consolidation means of the first type. The first excipient has a different solubility in the first liquid compared to the API and/or the second excipient such that the consolidation means of the first type may be suitable for only consolidating the first pattern.

In a further embodiment, the consolidation means of the second type may comprise a second printing liquid, different from the first printing liquid. The first excipient has a different solubility in the second printing liquid compared to the API and/or second excipient such that the consolidation of the consolidation means of the second type is suitable for only consolidating the second pattern.

In a further embodiment the first and the second printing liquids may comprise the same solvent but may have a different pH. A difference in pH between the first and the second printing liquid may be accomplished by providing a pH-modifier in the first or the second printing liquid.

In a preferred embodiment the consolidation means of the first type is suitable for an enteric coating

Using printing liquids may have the advantage of easily modifying the solubility of different components by changing the type of printing liquid like for example changing the pH of a solvent or by selecting different printing liquids. Furthermore, as the consolidation means of the first type is different from the consolidation means of the second type, the first pattern may comprise different material properties compared to the second pattern. The advantage of the consolidation means of the first type forming an enteric coating may be to ensure that the API is released after the stomach.

In another method according to the invention, the first and/or the second printing liquid may further comprise the active pharmaceutical ingredient or a further active pharmaceutical ingredient.

Including the API in the printing liquids may have the advantage of further increasing the loading of the active ingredient, in addition to the API already present in the tablet, such that the period of treatment may increase or the product may become more effective.

In a further method according to the invention, the consolidation means of the first type may comprise a first laser and optionally the consolidation means of the second type may comprise a second laser. The second laser may have a different wavelength than the first laser.

Working with lasers as consolidation means may have the benefit of expanding the means of consolidation by melting some of the components present in the homogeneous powder formulation.

Providing a second excipient in the homogeneous powder formulation, may increase the choice of API as the API may be selected independently from the consolidation of the second type.

In a further embodiment according to the invention, the method may further comprise of providing a first enteric coating, one or more pharmaceutical layers and a second enteric coating wherein the one or more pharmaceutical layer forms a core. The pharmaceutical layer may comprise a second formulation, optionally different from the homogeneous powder formulation. Additionally, the first and second enteric layer may comprise an enteric formulation, preferably different from the homogeneous powder formulation.

Providing a first enteric coating, followed by a pharmaceutical layer and a second enteric coating may have the advantage that the conventional process can be combined with a method according to the invention. Combining the conventional process with a method according to the invention may have the advantage of simplifying the overall manufacturing process of a tablet, preferably a pharmaceutical product.

Additionally, the present invention is aimed at a pharmaceutical product, preferably manufactured according to a method according to the invention. The pharmaceutical product comprises an enteric coating as outer contour. The enteric coating may be at least partially consolidated by the consolidation means of a first type. The enteric coating at least partially covers, preferably encapsulates a core including the active pharmaceutical ingredient. The core may comprise at least 30 wt.%, preferably at least 40 wt.%, more preferably at least 50 wt.% of the active pharmaceutical ingredient.

### BRIEF DESCRIPTION OF THE DRAWINGS

Figure 1A displays a homogeneous powder formulation.
Figure 1B depicts a homogeneous powder formulation consolidated by a consolidation means of the first type.
Figure 1C depicts a homogeneous powder formulation consolidated by a consolidation means of the second type.
Figure 2A displays a method of manufacturing a first and a second pattern of a tablet with a single process comprising two printing heads, printing liquids and a homogenous powder formulation.
Figure 2B depicts an expanded view of a pattern for producing a tablet according to the invention.
Figure 3 displays a pharmaceutical product according to the invention.
Figure 4 displays a homogeneous powder formulation including a second excipient consolidated by a consolidation means of the first type.
Figure 5A shows an intermediate tablet.
Figure 5B depicts a method of manufacturing a first pattern on the sides of tablet.

### DESCRIPTION OF EMBODIMENTS

For the purpose of clarity and a concise description, features are described herein as part of the same or separate embodiments, however, it will be appreciated that the scope of the invention may include embodiments having combination of all or some of the features described.

Terminology used for describing particular embodiments is not intended to limit the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise, it will be understood that when a connection between structures or components is described, this connection may be established directly or through intermediate structures or components unless specified otherwise.

As used herein the term "coated tablet", refers to any type of product wherein a core may be at least partially encapsulated by a coating protecting the core from environmental properties.

As used herein the term "pharmaceutical product" refers to a product that comprises core and an enteric coating. The core and/or the enteric coating comprises an active pharmaceutical ingredient.

At present, tablets are the still the preferred way to administer Active Pharmaceutical Ingredients (APIs), a drawback of various APIs may be that they may not be stabile under the conditions and environment like for example encountered in a stomach. Hence, protection from these conditions may be needed. Generally, tablets may be provided with an enteric coating, which often is composed of materials resilient to the acidic conditions and enzymes such as found in a stomach. These enteric coatings may be capable of dissolving within the gastrointestinal (GI) tract beyond the stomach. Typically, these enteric coatings may be put onto the pre-made tablets in a separate production step.

3D printing of tablets may offer great possibilities for multiple industries such as the pharmaceutical industry. In particular, the possibility to create personalized medication, e.g. tablets with a specific (personalized) dose of API, may be of great interest. Such personalization, presently, is difficult to achieve by means of conventional tableting processes.

When a tablet is 3D printed, the Active Pharmaceutical Ingredients (APIs) may need to be encapsulated with an enteric coating. Additionally, 3D-printed tablets may endure an additional coating step to achieve the desired characteristics. Due to the different surface characteristics of 3D printed tablets, providing an enteric coating may be more difficult to carry out.

Further to the aforementioned difficulties in creating an enteric coating, when feasible, on 3D printed tablets, the tablet production remains a multistep process. Accordingly, the present invention is aimed at a single step process to create both the tablet and the enteric coating would be beneficial.

A conventional 3D printing technique suitable for the manufacturing of a tablet, preferably a pharmaceutical product may be powder bed printing (PBP). Powder bed printing (PBP) is a 3D printing method that is suitable for joining powder formulations point by point using printing liquids as a consolidation method. Powder bed printing has the advantage that multiple materials like printing liquids and/or powder formulation, can be used in a single process. However, the disadvantage of powder formulations as a printing medium utilized during PBP may be related to the deposition of different formulations in different sections of a single layer as is required when producing a tablet preferably a pharmaceutical product. Even in the event that local deposition of multiple powder formulation would be possible, the expectation would be that such process would be slow and possibly economically not of interest. Further, a tablet manufactured with powder bed printing may result in a lower resolution. Additionally, the two formulations in a two-step process may reduce the recyclability of the non-used powders as both powders would be present in the powder bed.

Further, a PBP technique may comprise a polymeric material, preferably a polymeric solution as printing liquid, the polymeric material may act as an enteric layer. The drawback of using a polymer solution as a printing liquid may relate to the clogging or blocking of the nozzle during printing. Additionally, achieving a dense enteric coating may be difficult to achieve as the solubility of a polymer in a printing medium may be limited. Further, only a limited amount of printing liquid can be used before the powder formulation may be oversaturated. The low solubility of the polymer in the printing medium combined with the quick oversaturation may have a negative influence on the formation of a dense layer as is needed for an enteric coating. A dense coating encapsulating the pharmaceutical may be needed to ensure that a tablet may be able to resist specific conditions.

However, the ability of PBP to consolidate specific components by printing different printing liquids may also be exploited in a manner such that two different layers may be formed depending on the printing liquid used to consolidate a powder formulation. Accordingly, the present invention is intended to a method of manufacturing a tablet, preferably a pharmaceutical product that employs the versatility and the flexibility of powder bed printing.

The method for manufacturing a tablet, preferably a 3D printable pharmaceutical product comprises repeated steps of: providing a layer of a homogeneous powder formulation. The powder formulation comprises a first excipient and an active pharmaceutical ingredient (API). The first excipient suitable for consolidation by a consolidation means of a first type and the active pharmaceutical ingredient preferably not suitable for consolidation by the consolidation means of the first type. Once the homogeneous powder formulation is provided, the method is continued by applying the consolidation means of the first type in a first pattern on the layer of homogeneous powder formulation. The 3D printable pharmaceutical product having an outside contour formed by the first pattern such that an enteric coating is formed.

In a preferred embodiment according to the invention, the method may further comprise providing a second excipient suitable for consolidation by a consolidation means of a second type. Providing a second excipient suitable for consolidation by a consolidation means of the second type may have the advantage that an API can be selected independently from the consolidation means of the second type.

In another embodiment according to the invention, the second excipient and/or the API (3) may be suitable for consolidation by a consolidation of a second type (5).

In another embodiment, the method may further comprise the step of applying the consolidation means of the second type in a second pattern on the layer of homogeneous powder formulation. In a preferred embodiment the first and the second pattern may be complementary.

In a further embodiment the outside contour may act as an enteric coating. The outside contour as enteric coating may have a thickness of at least 0.05 mm, preferably at least 0.1 mm.

As the enteric coating may form when consolidating the homogeneous powder formulation also the API and optionally the second excipient are present in the enteric coating. Both the API as well as the second excipient may dissolve in the stomach when in contact with an aqueous solution. A core of a tablet that comprises the API is only appropriately protected by an enteric coating when no connected network is formed between the API and/or second excipient in the enteric coating wherein the enteric coating is consolidated by the consolidation means of the first type. The connected network may in specific scenarios form a pore such that the API present in the core may start to dissolve in the stomach. Therefore, a proper thickness of the enteric coating manufactured by the consolidation means of the first type may be required to ensure that the components such as the API and/or the second excipients do not dissolve while entering the stomach. Changing the consolidation means instead of the powder formulation may have the advantage of reducing the complexity of conventional two-step processes while increasing the flexibility and the versatility. Additionally, only one homogeneous powder formulation may be used throughout the production process such that it may be possible to produce a tablet in a single process. Further, the process may facilitate the switch to a more personalized medicine as a single process may reduce the overall cost of the tablet, making it economically more interesting. Additionally, the amount of API may be easily controlled as the geometry or size can be altered quickly.

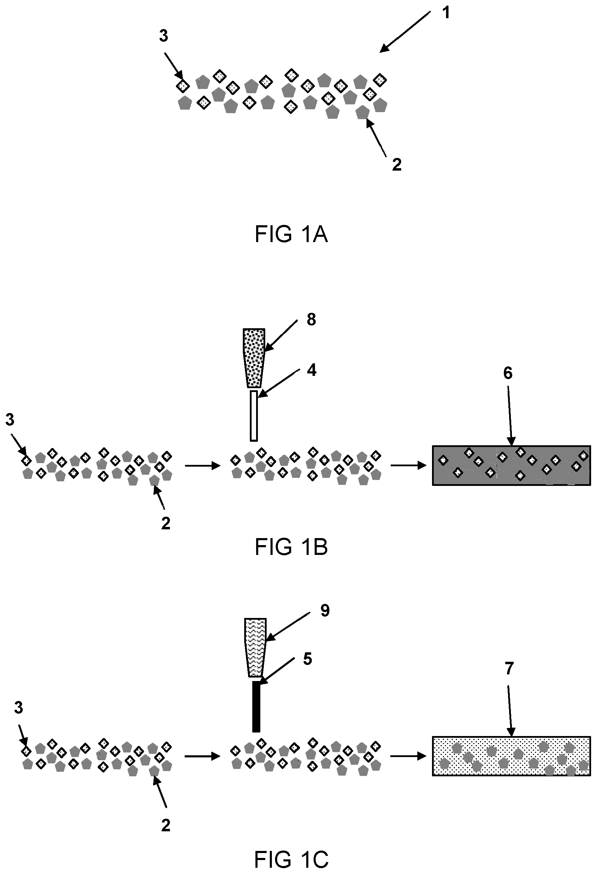

The homogeneous powder formulation comprises a first excipient, an API and optionally a second excipient. The first excipient and API may have different means of consolidation used to manufacture a tablet, preferably a pharmaceutical product as shown in Figure 1A.

Figure 1A displays a homogenous powder formulation 1 comprising an API 2 and an excipient 3. Typical APIs suitable for the method according to the invention may comprise any material known to date. Examples of APIs, without any limitation, include APIs from various pharmacological classes, e.g. antibacterial agents, antihistamines and decongestants, anti-inflammatory agents, antiparasitics, antivirals, local anaesthetics, antifungals, amoebicidals or trichomonocidal agents, analgesics, antianxiety agents, anticlotting agents, antiarthritics, antiasthmatics, anticoagulants, anticonvulsants, antidepressants, antidiabetics, antiglaucoma agents, antimalarials, antimicrobials, antineoplastics, antiobesity agents, antipsychotics, antihypertensives, autoimmune disorder agents, anti-impotence agents, anti-Parkinsonism agents, anti-Alzheimer's agents, antipyretics, anticholinergics, anti-ulcer agents, blood-glucose-lowering agents, bronchodilators, central nervous system agents, cardiovascular agents, cognitive enhancers, contraceptives, cholesterol-reducing agents, agents that act against dyslipidermia, cytostatics, diuretics, germicidials, H2 blockers, hormonal agents, anti-hormonical agents, hypnotic agents, inotropics, muscle relaxants, muscle contractants, physical energizers, sedatives, sympathomimetics, vasodilators, vasocontrictors, tranquilizers, electrolyte supplements, vitamins, uricosurics, cardiac glycosides, membrane efflux inhibitors, membrane transport protein inhibitors, expectorants, purgatives, contrast materials, radiopharmaceuticals, imaging agents, peptides, enzymes, growth factors, vaccines and mineral trace elements. Other API substances include proton pump inhibitors.

Figure 1B demonstrates how a consolidation by a consolidation means of the first type 4 may result in the consolidation of a first pattern 6 of a layer of the homogeneous powder formulation 1. The consolidation means of the first type is generated by a first consolidation means generator 8. The consolidation means generator 8 may any generator suitable of consolidating a component. Typical examples are a laser generator or print head comprising a printing liquid as developed in the further figures. The layer of homogeneous powder formulation 1 comprising an API 3 and a first excipient 2.

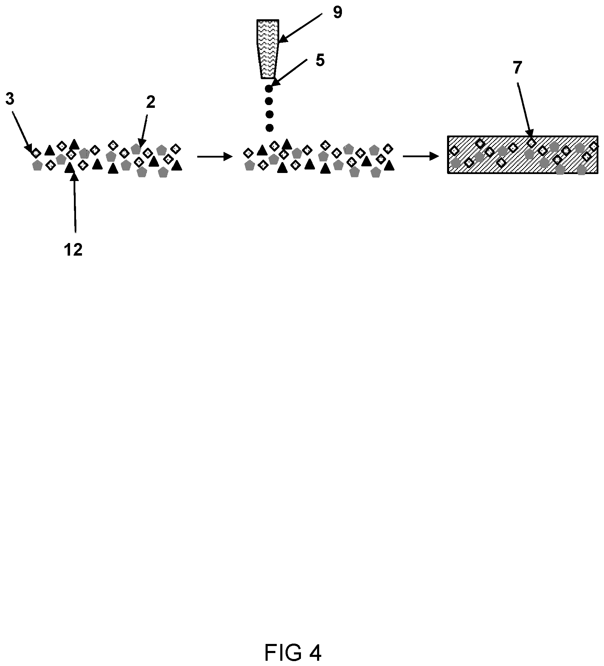

Figure 1C demonstrates how a consolidation of the second type 5 may result in the consolidation of a second pattern 7 of a layer of the homogeneous powder formulation 1. In figure 1C the layer of homogeneous powder formulation 1 comprises a first excipient 2 and an API 3 In figure 1C the API 3 is consolidated by the consolidation means of the second type 5 forming a second pattern 7. In figure 4 the homogeneous powder formulation comprises a first excipient 2, an API 3 and a second excipient 12.

In a preferred embodiment the 3D printed tablet, preferably a 3D printable pharmaceutical product may be manufactured in a single process.

A single process may have the advantage of increasing the efficiency of manufacturing a tablet. Further, it may benefit the ability to personalize medicine to the needs of a subject such as a patient.

In another embodiment according to the invention, the first and/or the second pattern may be fully consolidated.

A fully consolidated first and/or second pattern may allow the tablet to bypass highly acidic conditions like for example encountered in a stomach. Consequently, the API may be released in the gastrointestinal area after the stomach. Releasing the API in an appropriate location may increase the medicines effectiveness.

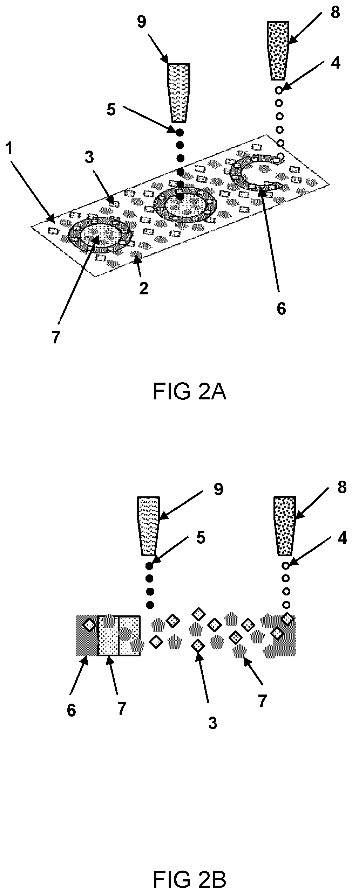

An example of single step process according to the invention is shown in figures 2A, and 2B. Figure 2A displays a method of manufacturing a tablet according to the invention. The method comprising a first printing head 8 comprising a consolidation means of the first type 4, a second printing head 9 comprising a consolidation means of the second type 5 and a homogeneous powder formulation 1.

In the shown embodiment, the consolidation means of the first type comprises a first printing liquid. The first excipient may have a different solubility in the first printing liquid compared to the API and/or second excipient present in the homogeneous powder formulation. The consolidation means of the first type may be suitable for only consolidating the first pattern.

In a further embodiment, the consolidation means of the second type may comprise a second printing liquid. The second printing liquid may be different from the first liquid. The first excipient may have a different solubility in the second printing liquid compared to the API and/or second excipient present in the homogeneous powder formulation. The consolidation means of the second type may be suitable for consolidating the second pattern.

In certain embodiments, the first and second printing liquids may comprise the same solvent and only differ in pH. A typical method of changing the pH of a solvent may be realized by including a pH-modifier in the first or second printing liquid. Other typical examples of the first and the second printing liquid may be a mixture of ethanol and water. The sole difference between the first and the second printing liquid may be the ratio of ethanol to water.

A pH-modifier may comprise any pH-modifier known to date suitable for modifying the pH of a solvent. pH modifiers may include acidifying, alkalizing, buffering agents, or mixtures thereof. Typical examples, but not limited to, comprise tricalicum phosphate, tannic acid, (S)-lactic acid, citric acid, (dilute) sulfuric acid, dipotassium hydrogen phosphate, disodium hydrogen phosphate, DL-tartaric acid, sodium carbonate, sodium citrate, or any combination thereof.

The homogeneous powder formulation may comprise powder particles having a particle size that may be in the range of 30 µm to 250 µm, preferably between 40 µm and 220 µm, more preferably between 80 µm and 200 µm. A to low particle size may result in a lower wettability of the particles.

First excipients suitable for forming an enteric coating may comprise any material known to date suitable for the manufacturing of an enteric coating that encapsulates a tablet, preferably a pharmaceutical product. Typical examples of first excipients suitable for forming an enteric coating may be methyl acrylate-methacrylic acid copolymers, cellulose acetate phthalate (CAP), cellulose acetate succinate, hydroxypropyl methyl cellulose phthalate, hydroxypropyl methyl cellulose acetate succinate (hypromellose acetate succinate), polyvinyl acetate phthalate (PVAP), methyl methacrylate-methacrylic acid copolymers, shellac, cellulose acetate trimellitate, sodium alginate, zein, or enteric coating aqueous solutions such as coated softgels (ethylcellulose, medium chain triglycerides like for example from coconut, oleic acid, sodium alginate, stearic acid), or mixtures thereof.

The homogeneous powder formulation comprising a first excipient 2 and an API 3. When the homogeneous powder formulation is printed by the consolidation means of the first type 4 (for example a first printing liquid 4), the first excipient 2 will dissolve into the printing liquid 4 forming a first pattern 6. The consolidation means of the first type 4 may be followed by a consolidation means of the second type 5 (for example a second printing liquid 5) such that the API may at least partially dissolve forming a second pattern 7. After printing the homogeneous powder formulation with a consolidation means of the first (4) and the second type (5), the homogeneous powder formulation may be dried such that the homogeneous powder formulation may further consolidate forming the final product.

Figure 2B displays an expanded view of a layer of homogeneous powder formulation 1 printed by consecutive printing heads 8, 9 comprising respectively the consolidation means of the first 4 and the second type 5. The expanded view, in particular shows how a first 6 and second pattern 7 can quickly be exchanged such that a tablet may be formed comprising an API and/or a second excipient encapsulated by an enteric coating.

Using different printing liquids may further increase the flexibility of manufacturing a tablet such as a pharmaceutical product as printing liquids can be interchanged or replaced rapidly.

Printing liquids suitable for dissolving a first excipient may be any material known to date. Conventional examples comprise, but are not limited to, acetone ethyl acetate, dichloromethane, tetrahydrofuran (THF), methyl acetate, ethanol, water, ethyl acetate, ethanol, methanol, acetic acid, aqueous alcohols, glycols, n-methyl pyrrolidone, n-hexane, diethyl ether, or mixtures thereof.

Common combinations between a first excipient and a printing liquid suitable for dissolving the enteric excipient may be: methyl acrylate-methacrylic acid copolymers with acetone, ethyl acetate or dichloromethane, cellulose acetate phthalate (CAP) with acetone, THF or methyl acetate, cellulose acetate succinate with acetone, or ethanol/dichloromethane, hydroxypropyl methyl cellulose phthalate with ethanol/water, acetone/ethanol or dichloromethane/ethanol, hydroxypropyl methyl cellulose acetate succinate (hypromellose acetate succinate) with ethanol/water, acetone/ethanol, or dichloromethane/ethanol, polyvinyl acetate phthalate (PVAP) with ethanol, or methanol, methyl methacrylate-methacrylic acid copolymers with THF, or ethyl acetate, shellac with alcohols like for example ethanol, or methanol, cellulose acetate trimellitate with THF, acetone, or dichloromethane, zein with acetic acid, aqueous alcohols, glycols, or acetone/water or lipids with n-methyl pyrrolidone, n-hexane, or diethyl ether.

Printing liquids suitable for at least partially dissolving the second excipient may be any material known to date. Typical examples of printing liquids suitable for at least partially dissolving the second excipient include lactose, sugars, dextrose, mannitol, dicalcium phosphate, maltodextrin, PVP, HPC, starch or derivates of it, dextrin, PVA, water, ethanol, or any combination thereof. Preferably the printing liquid for at least partially dissolving the second excipient is water, ethanol, or a combination thereof.

Printing liquids suitable for dissolving APIs may be any known material known to date, examples include any solvent that may easily evaporate such as methanol, acetone, ethyl acetate, toluene, propanol, isopropanol, pentane, hexane, acetonitrile, or any mixture thereof, preferably water, ethanol, or mixtures thereof.

In a further embodiment of the present invention, the first and/or the second printing liquid may comprise an active pharmaceutical ingredient in addition to the API present in the homogeneous powder formulation, preferably the API used in the homogeneous powder formulation.

Including the API in the printing liquid, in addition to the API present in the homogeneous powder formulation, may have the advantage of increasing the API load in the tablet such that the administration may have a longer effect on the patient.

In another embodiment according to the invention, the consolidation means of the first type may comprise a first laser and optionally the consolidation means of the second type may comprise a second laser. The second laser may have a different wavelength than the first laser.

Using laser may have the advantage to also utilize Selective Laser Sintering (SLS) as consolidation techniques and therefore may increase the selection of excipients used in the homogeneous powder formulation.

Typical materials may be any material known to date suitable for SLS and suitable for ingestion. Examples include, but not limited to, lipids, maltodextrins, sugars, polyvinylpyrrolidone (PVP), hydroxypropyl cellulose (HPC), polyethylene glycol (PEG) and derivates thereof, polymethacrylates, glycerol dibehenate, glyceryl distearate, glycerol Trimyristate, polyvinyl acetate, povidone, poly(ethylene glycol)-block-poly(propylene glycol)-block-poly(ethylene glycol), glycerol triester, polyethylene oxide (PEO), metacrylic acid-ethyl acrylate copolymer, polyvinyl alcohol (PVA), or any mixtures thereof.

The composition of the homogeneous powder composition may comprise at least 25 wt.% of a first excipient suitable for forming an enteric coating, preferably at least 40 w.%, more preferably at least 55 wt.%, even more preferably at least 70 wt.% based on the total weight of the homogeneous powder formulation. Additionally, the composition may comprise an API-concentration in the range of 30 - 75 wt.% and optionally a second excipient in a concentration of at least 10 wt.%, preferably at least 20 wt.% based on the total weight of the homogeneous powder formulation.

In a further embodiment, the method may further comprise the steps of providing a first enteric coating, one or more pharmaceutical layers and a second enteric coating wherein the one or more pharmaceutical layers forms a core. The one or more pharmaceutical layers may comprise a second formulation optionally different from the homogeneous powder formulation. The first and second enteric coating further may comprise an enteric formulation different from the homogeneous powder formulation. A tablet wherein the method may comprise the steps of providing a first and second enteric coating with one or more pharmaceutical layers would result in an intermediate product wherein the sides of a tablet would not include an enteric coating such that the intermediate product cannot be consumed by a subject. To consolidate the sides of the intermediate product, the method according to the invention may be used such that the homogeneous powder can be deposited at the sides of the intermediate product followed by consolidation by a consolidation means of the first type. In an even further embodiment the one or more pharmaceutical layer may be provided by the homogenous powder formulation such that sides of the intermediate product can be printed after depositing the one or more pharmaceutical layers. As such the method according to the invention may also be combined with more conventional techniques known in the art.

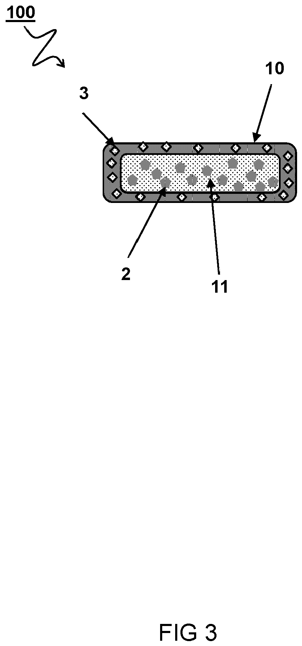

A further aspect of the present invention is aimed at a tablet, preferably a pharmaceutical product. The tablet preferably manufactured by a method according to the invention. The pharmaceutical product comprises an enteric coating as outer contour and a core, including the active pharmaceutical ingredient, encapsulated by the enteric coating. The enteric coating may be at least partially consolidated by the consolidation means of the first as shown in Figure 3.

Figure 3 depicts a tablet 100, preferably a pharmaceutical product comprising an enteric coating 10 and a core 11. The enteric coating 11 may be formed by a consolidation means of the first type. The core layer 10 may optionally be formed by the consolidation means of the second type.

Figure 4 displays a consolidation by consolidation means of the second type 5 wherein the second excipient 12 is dissolved forming a second pattern 7. The consolidation means of the second type generated by a second consolidation means generator 9. The consolidation means generator 9 may any generator suitable of consolidating a component.

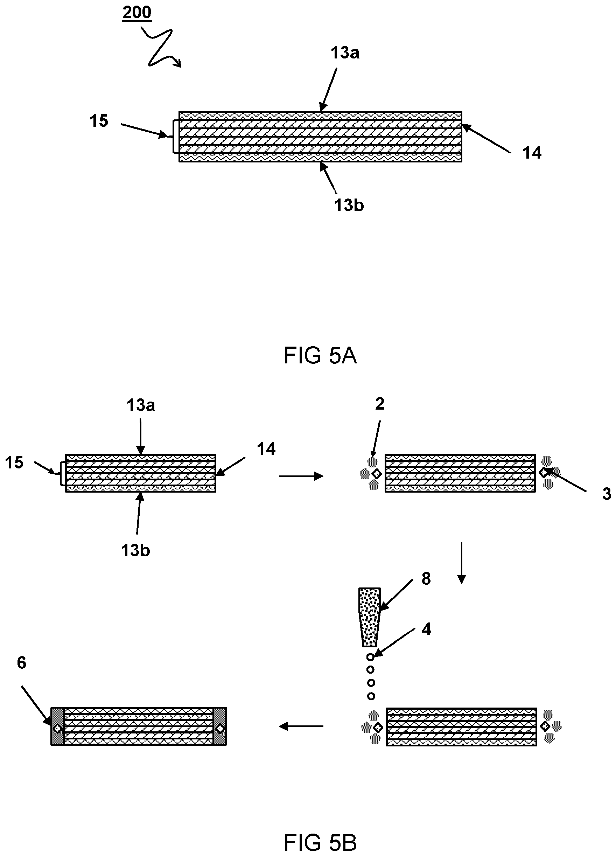

An example an intermediate product is shown in figure 5A. Figure 5A displays an intermediate product 200, the intermediate product 200 comprises a first enteric coating 13a and a second enteric coating 13b and one or more pharmaceutical layers 14 forming a core 15.

Accordingly there is shown
a. providing a first enteric coating 13a,
b. providing one or more pharmaceutical layers 14, the pharmaceutical layers 14 comprising a second formulation, optionally different from the homogeneous powder 1 formulation for forming a core 15;
c. providing a second enteric coating 13b;
wherein the first 13a and second enteric coating 13b comprise an enteric formulation preferably different from the homogeneous powder formulation (1).

Figure 5B displays a method according to the invention comprising the intermediate product 200. To manufacture a fully encapsulated tablet, the intermediate product 200 is further processed with the repeated steps of providing a homogeneous powder formulation 1 at both sides of the intermediate product 200. After providing the homogeneous powder formulation 1, the homogeneous powder formulation 1 is consolidated by a consolidation means of the first type 4, such as a printing liquid, forming a first pattern 6.

## Claims

1. A method for manufacturing a 3D printable pharmaceutical product (100), comprising repeated steps of:
a. providing a layer of a homogeneous powder formulation (1), the formulation comprising a first excipient (2) and an active pharmaceutical ingredient (3), the first excipient (2) suitable for consolidation by a consolidation means of a first type (4);
b. applying the consolidation means of the first type (4) in a first pattern (6) on the layer of homogeneous powder formulation (1);
wherein the first pattern (6) forms an outside contour of the 3D printable pharmaceutical product as an enteric coating.

2. The method according to claim 1, further comprises providing a second excipient not suitable for consolidation by the consolidation means of the first type (4) and suitable for consolidation by a consolidation means of a second type (5).

3. The method according to claim 1 of claim 2, wherein the API (3) is not suitable for consolidation by a consolidation of a first type (5).

4. The method according to any of claims 1-3, wherein the method further comprises applying the consolidation means of the second type (5) in a second pattern (7) on the layer of homogenous powder formulation (1).

5. The method according to claim 1, wherein the first (6) and second pattern (7) are complementary.

6. The method according to any of claims 1-5 , wherein the outside contour as enteric coating has a thickness of at least 0.05 mm, preferably at least 0.1 mm.

7. The method according to any of claims 1-6, wherein the 3D printable pharmaceutical product (100) is manufactured in a single process.

8. The method according to any of claims 1-7, wherein the first (6) and/or second pattern (7) is fully consolidated.

9. The method according to any of claims 1-8, wherein the consolidation means of the first type (4) comprises a first printing liquid and wherein the first excipient (2) has a different solubility in the first liquid compared to the API (3) and/or the second excipient, so that the consolidation means of the first (4) type is suitable for only consolidating the first pattern (6).

10. The method according to any of claims 1-9, wherein the consolidation means of the second type comprises a second printing liquid, different from the first printing liquid; and wherein the first excipient (2) has a different solubility in the second liquid compared to the API (3) and/or the second excipient, so that the consolidation means of the second type (5) is suitable for only consolidating the second pattern (6).

11. The method according any of claims 1-10, wherein the first and second printing liquid comprise the same solvent and a pH-modifier.

12. The method according to any of claims 1-11, wherein the first and/or the second printing liquid further comprises the active pharmaceutical ingredient (3) or a further active pharmaceutical ingredient.

13. The method according to any of claims 1-12, wherein the consolidation means of the first type (4) comprises a first laser and optionally the consolidation means of the second type (5) comprising a second laser having a different wavelength than the first laser.

14. The method according to any of the preceding claims, wherein the consolidation means of the first type (4) is an enteric coating.

15. A pharmaceutical product (100) manufactured according to any of the claims 1-14, the pharmaceutical product (100) comprising:
a. an enteric coating (10) as outer contour, wherein the enteric coating is at least partially consolidated by the consolidation means of the first type;
b. a core (11) including an API, encapsulated by the enteric coating.
